(19) Europäisches Patentamt
European Patent Office
Office européen des brevets

(11) EP 1 492 043 A2

# (12) EUROPEAN PATENT APPLICATION

(43) Date of publication:
**29.12.2004 Bulletin 2004/53**

(51) Int Cl.7: **G06F 19/00**

(21) Application number: **04014537.7**

(22) Date of filing: **22.06.2004**

(84) Designated Contracting States:
**AT BE BG CH CY CZ DE DK EE ES FI FR GB GR HU IE IT LI LU MC NL PL PT RO SE SI SK TR**
Designated Extension States:
**AL HR LT LV MK**

(30) Priority: **26.06.2003 US 483267 P**

(71) Applicant: **Accelrys Software, Inc.**
**San Diego California 92121 (US)**

(72) Inventors:
- **Sutter, Jonathan Mar**
  **Vista, California 92081 (US)**
- **Maynard, Allister J.**
  **La Jolla, California 92037 (US)**
- **Waldman, Marvin**
  **San Diego, California 92130 (US)**

(74) Representative:
**Sternagel, Fleischer, Godemeyer & Partner**
**Patentanwälte**
**Braunsberger Feld 29**
**51429 Bergisch Gladbach (DE)**

## (54) Pharmacophore model generation and use

(57) Methods and systems for generating pharmacophore models are characterized both by molecular features that are present in the model and features that are defined as absent. Thus, models can be developed that take into account features such as steric bulk that inhibit activity for a specified target as well as features such as functional groups that promote activity. Features that inhibit activity can be identified by comparing known active molecules with known inactive molecules. Features that are present in the inactive molecules but absent in the active molecules can be defined in a pharmacophore model.

EP 1 492 043 A2

## Description

The present invention relates to computational chemistry. More specifically, the present invention relates to the generation and use of pharmacophore models.

Several computational methods are available for researchers to use in predicting the activities and/or experimental properties of molecules. Some of these methods include the generation of one or more pharmacophores. Pharmacophores are 3-dimensional representations of features of molecules that correlate with a specified activity/ property, e.g. a Hydrogen bond donor at location A, a hydrophobic group at location B, etc. Once a pharmacophore corresponding to a desired activity/property is defined, one or more molecules can be screened for the activity/property by determining which of the screened molecules have features that significantly overlap the features defined by the pharmacophore. An overview of pharmacophore definition and pharmacophore directed database searching is provided in Greene et. al, Chemical Function Queries for 3D Database Search, *J. Chem. Inf. Comput. Sci.* 34, 1297-1308 (1994).

The earliest pharmacophores were manually developed from direct researcher study of 3D structures of ligands and/or associated binding sites in an attempt to understand the most important features of the binding mechanism. One such example is a CNS pharmacophore described in Lloyd et. al, A Common Structural Model for Central Nervous System Drugs and their Receptors, *J. Med. Chem. 29* 453-462 (1986).

In the late 1980s, a program known as DISCO was created which attempted to automate the process of defining pharmacophores that successfully correlate structural and/or functional molecular features with activity. This program performed an automated search over a set of active compounds for common structural and/or functional features positioned in similar spatial relationships.

One aspect of pharmacophore generation that has received some attention has been the attempt to include "excluded volumes" in the pharmacophore definition. This issue arises because a particular molecule may contain the structural and/or functional features required for activity, but some portion of the molecule may be located relative to the necessary functional features such that steric interference prevents binding to the target. Thus, it has been found useful to define regions around the activity producing features of the pharmacophore which are not allowed to contain atoms.

In cases where the structure of the target binding pocket is known, improved pharmacophores have been developed by choosing an excluded volume region corresponding to the inner surface of the binding pocket. See, for example, Chapters 18 and 20 of "Pharmacophore Perception, Development, and Use in Drug Design," edited by Osman F. Güner, International University Line, ISBN 0-9636817-6-1 (2000). In addition, the pharmacophore generating program ALLADIN allowed the user to define a point grid or set of spheres defining excluded volume regions of pharmacophores.

Although excluded volumes have been incorporated into pharmacophores, there remains a significant amount of user interaction required to successfully incorporate them. Furthermore, in many cases, no binding pocket structure information is available.

Thus, the object of the present invention is to provide better methods of defining excluded volume regions, especially methods that allow more automated excluded volume definition.

This object is met by the methods and the embodiments claimed in the attached claims and described herein.

One embodiment is a method of defining a pharmacophore comprising: defining a first location as exhibiting a first selected molecular feature; and defining a second location as lacking a second selected molecular feature, wherein the second location is determined by: 1) aligning a first molecule that exhibits an activity against one or more targets to an initial version of a pharmacophore; 2) aligning a second molecule that exhibits less activity against the one or more targets to the initial version; and 3) identifying as the second location a molecular feature of the second molecule that is inconsistent with one or more molecular features of the first molecule.

Another embodiment is a method of defining a pharmacophore comprising: defining a first location as exhibiting a first selected molecular feature; and defining a second location as lacking a second selected molecular feature, wherein the second location is determined by: 1) aligning a first molecule that exhibits an activity against one or more targets to a second molecule that exhibits less activity against the one or more targets; and 2) identifying as the second location a molecular feature of the second molecule that is inconsistent with one or more molecular features of the first molecule.

Another embodiment is a method of defining a feature as absent in a pharmacophore comprising: aligning a first molecule that exhibits an activity against one or more targets to a second molecule that exhibits less activity against the one or more targets; and identifying as the feature a molecular feature of the second molecule that is inconsistent with one or more molecular features of the first molecule.

Another embodiment is a method of defining a feature as absent in a pharmacophore comprising: aligning a molecule that is inactive against one or more targets to an initial version of the pharmacophore; and identifying as the feature a molecular feature of the molecule that is inconsistent with one or more molecular features of the initial version.

Another embodiment is a method of optimizing a pharmacophore model of a molecular entity expected to have activity against one or more targets; the method comprising: aligning a first molecule that exhibits the activity against the target with an initial version of the pharmacophore model; aligning a second molecule that does not exhibit the activity against the target with the initial version of the pharmacophore model; identifying a molecular feature of the second molecule that is inconsistent with the molecular features of the first molecule when both are aligned with the pharmacophore model; and updating the pharmacophore model to include a requirement that the identified molecular feature be absent.

Another embodiment is a method of defining a pharmacophore model of a molecule exhibiting a particular property, the method comprising defining a first set of molecular features as present and a second set of molecular features as absent, wherein the presence of the second set of molecular features in a molecule inhibits the molecule from exhibiting the property, the second set of molecular features determined by comparing a molecule exhibiting the particular property with a molecule not exhibiting the particular property.

Another embodiment is a method of estimating the activity of a molecule comprising: increasing the estimated activity if a molecular feature of the molecule is within a specified distance from a corresponding feature defined as present in a pharmacophore model; and decreasing the estimated activity if a molecular feature of the molecule is within a specified distance from a region defined as excluded in the pharmacophore model.

Another embodiment is an *in silico* molecular screening system comprising: a memory having stored therein a pharmacophore model of molecules predicted to exhibit a particular property, wherein the pharmacophore model defines one or more molecular features and their respective spatial positions as absent; and a processor configured to compare candidate molecules to the pharmacophore model by aligning the candidate molecules with the pharmacophore model and determining whether or not the one or more molecular features are present in the candidate molecules.

Another embodiment is a system for generating a pharmacophore for use in molecular screening comprising: a memory storing molecular structures of a set of training molecules for which activity is known; a pharmacophore generation module configured to generate a pharmacophore model and store the model in the memory; the pharmacophore generation module comprising an active molecular feature presence module and an inactive molecular feature presence module, wherein the active molecular feature presence module defines molecular features for inclusion in the pharmacophore whose presence contributes to activity and the inactive molecular feature presence module defines molecular features to be designated in the pharmacophore as absent whose presence inhibits activity, wherein molecular features to be designated as absent are determined by aligning two molecular structures in the training set that have different activities and identifying a molecular feature in one of the two molecular structures that is inconsistent with one or more molecular features in the other molecular structure; a molecule-pharmacophore comparison module configured to retrieve a molecular structure in the training set and the pharmacophore from the memory and determine similarity between the molecular structure and the pharmacophore; and an activity-prediction module configured to estimate activity of the molecule corresponding to the molecular structure based on the similarity, wherein the estimated activity is used by the pharmacophore generation module in generating the pharmacophore model.

Another embodiment is a system for estimating activity of a test molecule comprising: a memory storing a pharmacophore model and a molecular structure of the test molecule; a molecule-pharmacophore comparison module configured to retrieve the pharmacophore model and the molecular structure from memory and determine similarity between the molecular structure and the pharmacophore, wherein the similarity is based on molecular features that are defined as present in the pharmacophore and molecular features that are defined as absent in the pharmacophore; and an activity prediction module configured to estimate activity of the molecule based on the similarity, wherein the estimated activity is decreased if the molecule contains the molecular features that are defined as absent.

As discussed above, pharmacophores based entirely on what must be *included* in the model ignores contributions to inactivity caused by molecular features present in the molecules of the training set that do not have the desired activity or properties. Thus, there is a need for algorithms that generate and make use of pharmacophores that are constructed based not only on what molecular features must be included, but also what features must be *absent*.

In some embodiments, an algorithm is provided that defines a pharmacophore that is at least partially characterized by the absence of some molecular structure or feature. One example of the utility of such a pharmacophore is when there is an incompatibility between a molecule's shape (steric bulk) and the shape of a molecular target. For example, while a particular molecule may have functional groups that generally characterize a class of molecules as active for some target, the molecule may have additional steric bulk that prohibit the molecule from successfully binding with the target. A pharmacophore that is defined by both the presence of the functional groups and the absence of steric bulk in selected regions enhances the accuracy of molecular activity/property predictions using the pharmacophore.

Pharmacophore Model Generation

General aspects of pharmacophore generation and use are described in "Pharmacophore Perception, Development, and Use" by Osman F. Güner, International University Line. Generally, pharmacophore generation involves iterative improvement of one or more candidate pharmacophores. This general pharmacophore generation method has been incorporated into the program CATALYST, developed and marketed by Accelrys Software of San Diego, California. This program begins with a "constructive phase" that looks for common feature arrangements in one or more of the most active molecules in a training set of molecules for which numerical activity data is known. It then uses a "subtractive phase" where common feature arrangements found in the constructive phase are eliminated from further consideration if they also cover too many of the less active molecules of the training set. Finally, the pharmacophore candidates that survive the constructive and subtractive phases are perturbed in an "optimization phase." In this process, the definition of a pharmacophore is perturbed or "moved" and the effect of the "move" on pharmacophore predictive accuracy with respect to the training set of molecules is determined. In one embodiment, if the move improves predictive accuracy, the move is accepted. If the move does not improve predictive accuracy, it may be accepted or rejected based on a variety of possible known Monte Carlo simulation decision criteria. These processes are described in more detail in Chapters 10 and 26 of Güner, *supra*.

In one embodiment, the general outline of this optimization process is illustrated in Figure 1. The model is designed to be predictive of molecular activity. At block 2 a training set of molecules is provided. The training set typically includes molecules that are classified as active and molecules that are classified as inactive. The activity of the molecules in the training set are determined such as by experimental assays of the molecules. The activity of a molecule is conventionally defined as the molar concentration of the molecule required to bind to 50% of the target in solution ($IC_{50}$), or as the -log($IC_{50}$) with the $IC_{50}$ typically being in the nanomolar to millimolar range. Thus, a molecule with less binding affinity for the target has a larger $IC_{50}$ and a smaller - log($IC_{50}$). Because the terms "active" and "inactive" are relative, the classification of a molecule to either category can be made in a variety of ways. For example, one or more of the molecules having the lowest $IC_{50}$ (highest -log($IC_{50}$)) may be defined as "active" and the remaining molecules may be defined as "inactive." As another alternative, an "inactive" molecule of the training set may defined as one that has an $IC_{50}$ of a certain threshold amount greater than the molecule of the training set with the lowest $IC_{50}$.

At block 4, an initial pharmacophore is selected. In the CATALYST program, this is done in the constructive and subtractive phases, but any method of generating a pharmacophore candidate may be used. In one embodiment, the initial pharmacophore is based on a selection of one of the molecules in the training set that has high activity. In one embodiment, the entire molecular structure of the selected molecule is used as the initial pharmacophore. In other embodiments, a subset of the molecular structure is used, such as by removing all hydrogen atoms. In still other embodiments, only specified functional groups present in the selected molecule are included in the initial pharmacophore.

At block 6, a "move" is selected to perturb the pharmacophore. In some embodiments, the "move" includes adding or removing a functional group or atom. In other embodiments, functional groups or atoms in the pharmacophore are translated. In advantageous embodiments, the "move" may also includes adding, removing, or translating features that must be absent. These features can include steric bulk or charged atoms. At block 8, the "move" is performed on the pharmacophore to change it. At block 10, predicted activities for the molecules in the training set are calculated by comparing the pharmacophore with each molecule. At block 12, the predicted activities are compared with the known activities to determine the predictive accuracy of the pharmacophore. In some embodiments, this comparison comprises calculating a cost as defined in the above mentioned Chapter 26 of Güner.

At decision block 14, it is determined whether or not to accept the "move" performed on the pharmacophore. In some embodiments, the "move" is accepted if it improves the predictive accuracy of the pharmacophore. In some embodiments, the "move" is accepted even if it does not improve predictive accuracy as long as it meets a MC Metropolis acceptance criterion. In some embodiments, determination of whether to accept the "move" is based on the change in cost. For example, a simulated anneal function of the change in cost may be used as the acceptance criterion. If the "move" is rejected, it is undone at block 16 and the algorithm proceeds at block 18. If the "move" is accepted, the algorithm immediately advances to block 18.

At decision block 18, it is determined whether a specified convergence criterion is met. In some embodiments, the convergence criterion is based on the predictive accuracy reaching a specified threshold. In other embodiments, the convergence criterion is based on there being no significant improvement in predictive accuracy with additional "moves." In still other embodiments, the convergence criterion is based on the improvement in predictive accuracy dropping below a specified threshold. If the convergence criterion is met, the algorithm stops at block 20 and the resulting pharmacophore can be used to predict the activity of molecules for which the activity is unknown. If the convergence criterion is not met, the algorithm returns to block 6 to select another "move" in an attempt to further improve the predictive accuracy of the pharmacophore.

In some embodiments, the *n* top pharmacophores are stored as the optimization algorithm operates. For

example, after each accepted "move," the algorithm can determine whether the new pharmacophore has better predictive accuracy than the worst pharmacophore currently stored in the top list. If the predictive activity is better, the new pharmacophore is added to the list and the worst pharmacophore on the list is discarded. Upon completion of the algorithm, one or more pharmacophores from the top list can be selected for use in predicting activities of molecules whose activities are not known.

Determination of Excluded Volume

In some embodiments, a "move" for adding excluded volume (absence of steric bulk) to a pharmacophore is determined by aligning one of the most active molecules in the training set to one of the inactive molecules in the training set. In one embodiment, the two molecules are also aligned to the current pharmacophore. Any atoms in the aligned inactive molecule that are greater than a threshold distance from all the atoms in the aligned active molecule could be responsible for the low activity of the inactive. The locations of these atoms may thus be used as candidate locations for adding excluded volumes. In another embodiment, only one less active molecule is aligned to the current pharmacophore. Atoms in the aligned molecule that are greater than a threshold distance from features defined as present in the pharmacophore are used as candidate locations for adding excluded volume. Adding an excluded volume will decrease the predicted activity of some molecules whose atoms encroach on the excluded volumes.

An algorithm for determining excluded volume is illustrated in the flowchart of Figure 2. At block 50, the molecules in the training set are classified as being active or inactive. In one embodiment, the classification is user defined. In another embodiment, the classification is determined based on those molecules having an activity above or below some threshold. In one embodiment, inactive molecules are defined as those molecules for which the following criterion is met:

$$\log(IC_{50}\ \textit{of candidate}\ \text{inactive molecule}) - \log(IC_{50}\ \text{of the most}\ \textit{active}\ \text{compound}) > \textit{threshold}$$

where *threshold* is user defined and has a default value of 3.5.

At block 52, the inactive molecule having the highest fit score to the currently hypothesized pharmacophore is selected. The fit score may be determined as described below. At block 54, one or more molecules in the training set that are classified as active are aligned to the pharmacophore. A procedure for aligning a molecule and a pharmacophore is described below. The co-ordinates of the atoms of the active molecules are used to create an active atom list. In one embodiment, the excluded volume algorithm is only pursued if the active molecules have an alignment fit score to the pharmacophore greater than the fit score of the selected inactive molecule. At block 56, the selected inactive molecule is aligned with the pharmacophore. The co-ordinates of the inactive molecule are used to generate an inactive atom list. In some embodiments, only specified atoms are included in the active and inactive atom lists. For example, only non-hydrogen atoms may be included. At block 58, the atoms in the inactive atom list that are further than a threshold distance from all of the atoms in the active atoms list are identified. In some embodiments, the threshold distance is user selected. In some embodiments, the threshold distance has a default value of 1.2 Angstroms. At block 60, one or more excluded volume locations are selected from the locations of the atoms identified at block 58. In some embodiments, the locations are randomly selected from the identified atoms. Finally, at block 62, excluded volume is added to the pharmacophore. In some embodiments, the excluded volume is defined as a sphere centered on the locations selected in block 58 having a specified radius. In some embodiments, the radius is 1.2 Angstroms.

Once excluded volume is added to a pharmacophore, later "moves" may remove the one or more excluded volumes or translate them to other locations.

Alignment of Molecules

Molecules may be aligned to one another and/or to a pharmacophore using a variety of currently known methods. A large body of literature describes such methods, many of which have been incorporated into commercially available products such as DISCO and CATALYST. Alignment of two molecules may be performed, for example, by aligning both to a common pharmacophore. Alignment of a molecule to a pharmacophore may be performed by defining a fit value that characterizes the overlap between a molecule and a pharmacophore. In some embodiments, the fit value is characterized by both alignment of features that the pharmacophore designates as being present and features that the pharmacophore designates as being absent. In some embodiments, features in the pharmacophore are assigned weight values that indicate their relative importance in the pharmacophore model as described in Chapter 26 of Güner, *supra*. In some embodiments, a default weight of 1.0 is assigned to all features.

Each feature of a pharmacophore may be defined by one or more location constraints that specify 3-dimensional coordinates. Furthermore, each location constraint may have associated with it a sphere of specified radius that defines a tolerance about each location constraint.

In some embodiments, the fit value is determined by the following formula:

$$fit = \sum_i weight(f_i)[1 - SSE(f_i)]$$

where each $f_i$ is a feature that is present in the pharmacophore, $weight(f_i)$ is the weight assigned to the $i$-th feature, and $SSE(f_i)$ is defined by:

$$SSE(f_i) = k\sum_j \left(\frac{D(c_{i,j})}{T(c_{i,j})}\right)^2$$

where each feature $f_i$ has $j$ location constraints, which can be different for each feature, $c_{ij}$ are the location constraints for each feature $f_i$, $D(c_{i,j})$ is the displacement of atom positions in the test molecule from the corresponding centers of location constraints $c_{i,j}$ in feature $f_i$, and $T(c_{i,j})$ is the radius of the location constraint sphere (tolerance) and k may be either 1 or 1/j. In some embodiments, a test molecule and the pharmacophore are aligned by finding the position and orientation of the molecule that maximizes *fit*. Any of the many fitting algorithms known in the art may be used in maximizing *fit*.

The above-indicated *fit* value may be adjusted to take into account features that are defined as being absent in the pharmacophore. For example, if a pharmacophore contains an excluded volume, the fit score may be left unaffected if the molecule being tested against the pharmacophore does not have any atom vdW (van der Waals) volume inside the excluded region. The fit may be defined as zero if the test molecule includes an atomic vdW volume inside the excluded region. Alternatively, a defined amount of overlap between the excluded volume and an atomic vdW volume of the test molecule may be allowed. In this case, the fit score may be scaled by an amount that is dependent on the amount of overlap. In some embodiments, hydrogen atoms are ignored in adjusting the *fit* value for overlap with an excluded volume.

In one implementation of adjusting the *fit* value for an excluded volume, a distance $d$ between an atom in the test molecule and the center of the excluded volume may be determined. If $d < xt$, where $x$ is a specified excluded volume factor and $t$ is the tolerance (radius) of the excluded volume plus the van der Waals radius of the atom, then *fit* is adjusted to be zero because an atom of the test molecule is within the excluded volume. If $xt < d < t$, then *fit* may be multiplied by:

$$\left(\frac{d - xt}{t - xt}\right)^2$$

to account for allowed overlap between an atom and the excluded volume. *If* $d > t$, *fit* may be left unchanged because the atom is not within an excluded volume of the pharmacophore. Other criteria and adjustment schemes to account for molecular features that are defined as absent in the pharmacophore may also be used.

Calculation of Predicted Activities

As discussed above, the activity of a molecule can be predicted by comparing it with a model pharmacophore. Such prediction may be used with training molecules as part of the process of generating an optimized pharmacophore as described above or to predict the activity of a molecule for which activity is not known. In some embodiments, the predicted activity is calculated by determining the similarity between the test molecule and the pharmacophore such as by the methods described above. Higher similarity between the test molecule and the pharmacophore leads to a higher predicted activity. In one embodiment, activity is estimated by the following formula:

$$activty = 10\ \exp[-(fit + intercept)]$$

Where *activity* is the predicted $IC_{50}$ for the molecule, *fit* is as defined above and *intercept* is determined using a regression analysis to maximize correlation between predicted activities and actual activities of the training set of molecules.

Pharmacophore System

The algorithms described above may be implemented in a general purpose computer system comprising a memory and a processor. One such embodiment is depicted in Figure 3. The system of Figure 3 comprises a memory 100. The memory 100 can be used to store one or more pharmacophore models as well as the molecular structures of one or more training molecules and/or one or more test molecules. Pharmacophore generation module 102 operates to retrieve the structures of training molecules from memory 100 and construct an optimized pharmacophore. The pharmacophore generation module 102 comprises an active molecular feature presence module 104 that determines features that are to be included in the pharmacophore. The pharmacophore generation module 102 also comprises an inactive molecular feature presence module 106 that determines features such as excluded volumes that are defined as absent in the pharmacophore. In making its determinations, the pharmacophore generation module 102 makes use of a molecule-pharmacophore comparison module 108 that determines the similarity between the training set molecules and a pharmacophore. The pharmacophore generation module 102 can also make use of an activity prediction module 110 that calculates predicted activity of the training set molecules based on the results produced by the molecule-pharmacophore comparison module 108.

The activity prediction module 110 can also be used to predict the activity molecules for which activity is unknown. In this embodiment, the molecule-pharmacophore comparison module 108 determines the similarity between the molecule and a pharmacophore, whose structures are stored in memory 100. The activity prediction module can then make use of this determination to calculate predicted activity.

The above described algorithms have several advantages. One is that excluded volumes which improve pharmacophore predictive accuracy can be defined in an automated way without extensive user interaction or knowledge of target binding sites. It is another advantage that the methods can be extended to incorporate additional definitions of features defined as absent in a pharmacophore model. For example, instead of excluded volumes, inactive molecules could be aligned with active molecules and/or a pharmacophore candidate and be screened for the presence of other specific features such as charged regions, certain functional groups, or specific atom types that may also interfere with binding affinity. These other types of features could then be tested as part of pharmacophore generation in the above described pharmacophore optimization process. This significantly extends the flexibility of pharmacophore generation from methods used previously.

Figures

FIGURE 1 is a flowchart illustrating an algorithm for generating pharmacophore models.

FIGURE 2 is a flowchart illustrating an algorithm defining excluded volumes in a pharmacophore model.

FIGURE 3 illustrates a system for generating and using pharmacophore models.

## Claims

1. A method of defining a pharmacophore comprising:

    defining a first location as exhibiting a first selected molecular feature; and
    defining a second location as lacking a second selected molecular feature, wherein said second location is determined by:

        aligning a first molecule that exhibits an activity against one or more targets to an initial version of a pharmacophore;
        aligning a second molecule that exhibits less activity against said one or more targets to said initial version; and
        identifying as said second location a molecular feature of said second molecule that is inconsistent with one or more molecular features of said first molecule.

2. The method of Claim 1, wherein said second selected molecular feature comprises steric bulk.

3. The method of Claim 1 or 2, wherein said second molecular feature comprises a selected atomic functional group.

4. The method of any of Claims 1 to 3, wherein said second molecular feature comprises a charged moiety.

5. The method of any of Claims 1 to 4, wherein said second molecular feature comprises a selected atom type.

6. The method of Claim 5, wherein said second molecular feature comprises a selected set of atom types.

7. A method of defining a pharmacophore comprising:

    defining a first location as exhibiting a first selected molecular feature; and
    defining a second location as lacking a second selected molecular feature, wherein said second location is determined by:

        aligning a first molecule that exhibits an activity against one or more targets to a second molecule that exhibits less activity against said one or more targets; and
        identifying as said second location a molecular feature of said second molecule that is inconsistent with one or more molecular features of said first molecule.

8. A method of defining a feature as absent in a pharmacophore comprising:

    aligning a first molecule that exhibits an activity against one or more targets to a second molecule that exhibits less activity against said one or more targets; and
    identifying as said feature a molecular feature of said second molecule that is inconsistent with one or more molecular features of said first molecule.

9. A method of defining a feature as absent in a pharmacophore comprising:

    aligning a molecule that is inactive against one or more targets to an initial version of said pharmacophore; and
    identifying as said feature a molecular feature of said molecule that is inconsistent with one or more molecular features of said initial version.

10. A method of optimizing a pharmacophore model of a molecular entity expected to have activity against one or more targets; said method comprising:

    aligning a first molecule that exhibits said activity against said target with an initial version of said pharmacophore model;
    aligning a second molecule that does not exhibit said activity against said target with said initial version of said pharmacophore model;
    identifying a molecular feature of said second molecule that is inconsistent with the molecular features of said first molecule when both are aligned with said pharmacophore model; and
    updating said pharmacophore model to include a requirement that said identified molecular feature be absent.

11. A method of defining a pharmacophore model of a molecule exhibiting a particular property, said method comprising defining a first set of molecular features as present and a second set of molecular features as absent, wherein the presence of the second set of molecular features in a molecule inhibits the molecule from exhibiting said property, wherein said second set of molecular features is determined by comparing a molecule exhibiting the particular property with a molecule not exhibiting the particular property.

12. A system for generating a pharmacophore for use in molecular screening comprising:

    a memory storing molecular structures of a set of training molecules for which activity is known;
    a pharmacophore generation module configured to generate a pharmacophore model and store said model in said memory; the pharmacophore generation module comprising an active molecular feature presence module and an inactive molecular feature presence module, wherein said active molecular feature presence module defines molecular features for inclusion in said pharmacophore whose presence contributes to activity and said inactive molecular feature presence module defines molecular features to be designated in said pharmacophore as absent whose presence inhibits activity, wherein molecular features to be designated as absent are determined by aligning two molecular structures in said training set that have different activities and identifying a molecular feature in one of the two molecular structures that is inconsistent with one or more

molecular features in the other molecular structure;
a molecule-pharmacophore comparison module configured to retrieve a molecular structure in said training set and said pharmacophore from said memory and determine similarity between said molecular structure and said pharmacophore; and
an activity-prediction module configured to estimate activity of the molecule corresponding to said molecular structure based on said similarity, wherein said estimated activity is used by said pharmacophore generation module in generating said pharmacophore model.

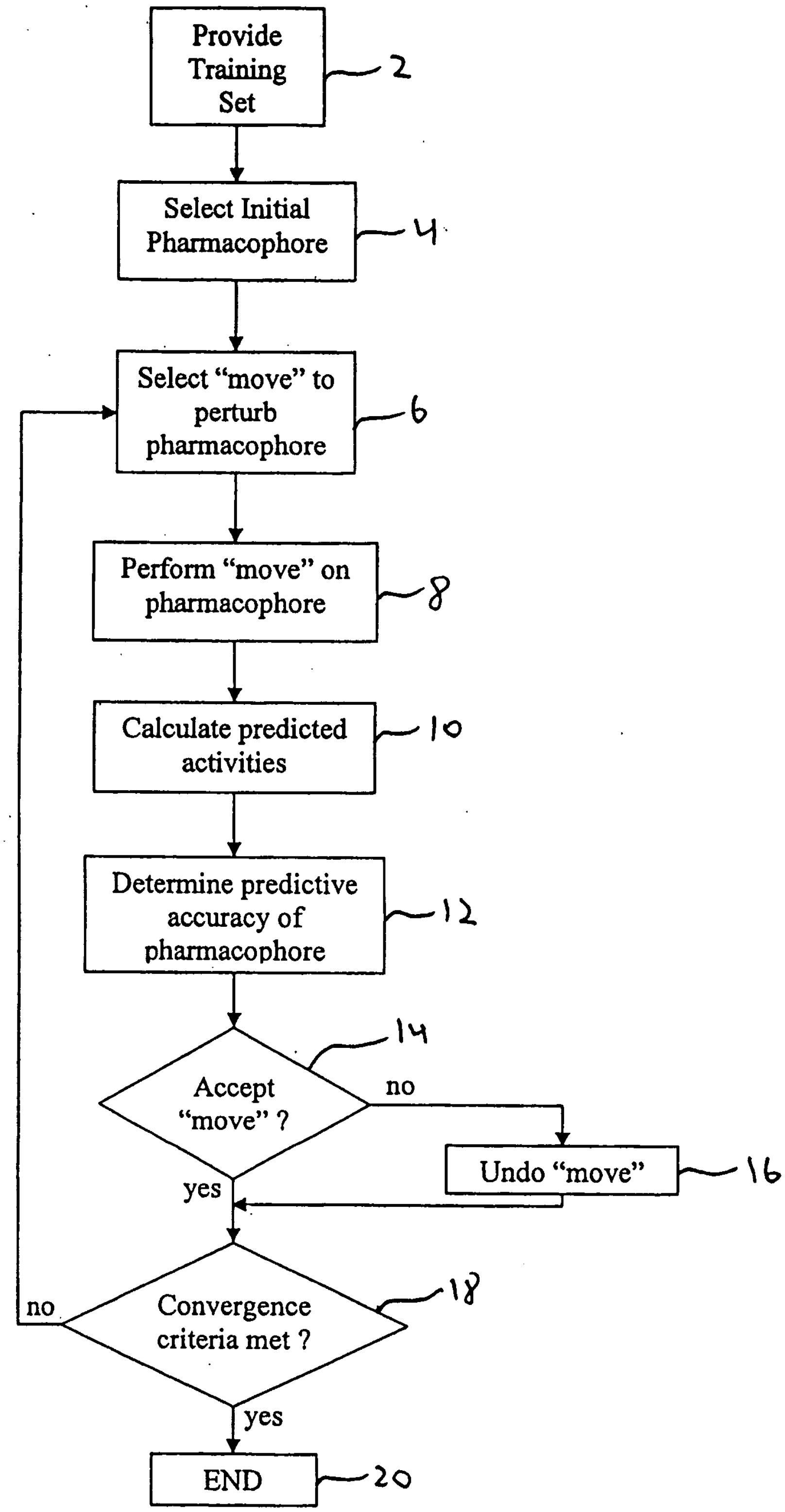
Provide Training Set
2
Select Initial Pharmacophore
4
Select "move" to perturb pharmacophore
6
Perform "move" on pharmacophore
8
Calculate predicted activities
10
Determine predictive accuracy of pharmacophore
12
Accept "move" ?
14
no
Undo "move"
16
yes
Convergence criteria met ?
18
no
yes
END
20

FIG. 1

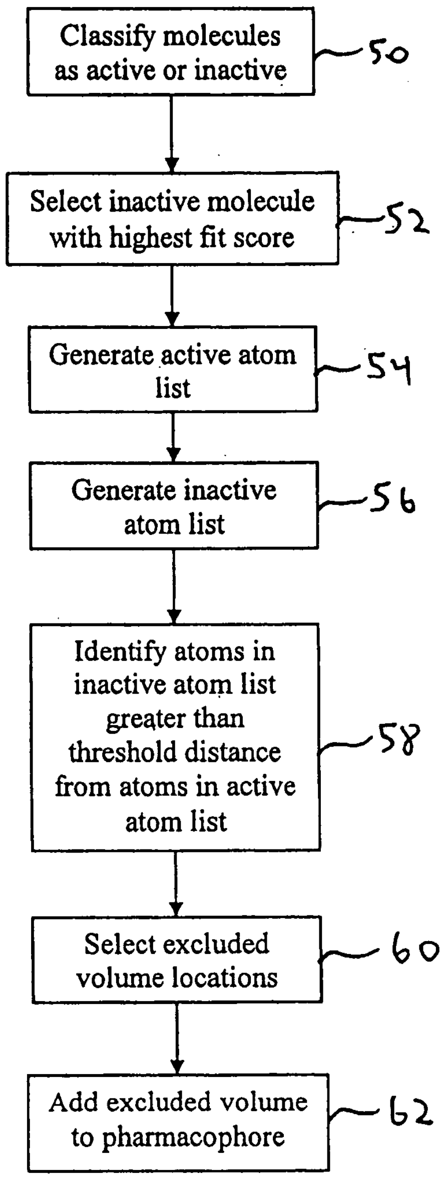
Classify molecules as active or inactive
50
Select inactive molecule with highest fit score
52
Generate active atom list
54
Generate inactive atom list
56
Identify atoms in inactive atom list greater than threshold distance from atoms in active atom list
58
Select excluded volume locations
60
Add excluded volume to pharmacophore
62

FIG. 2

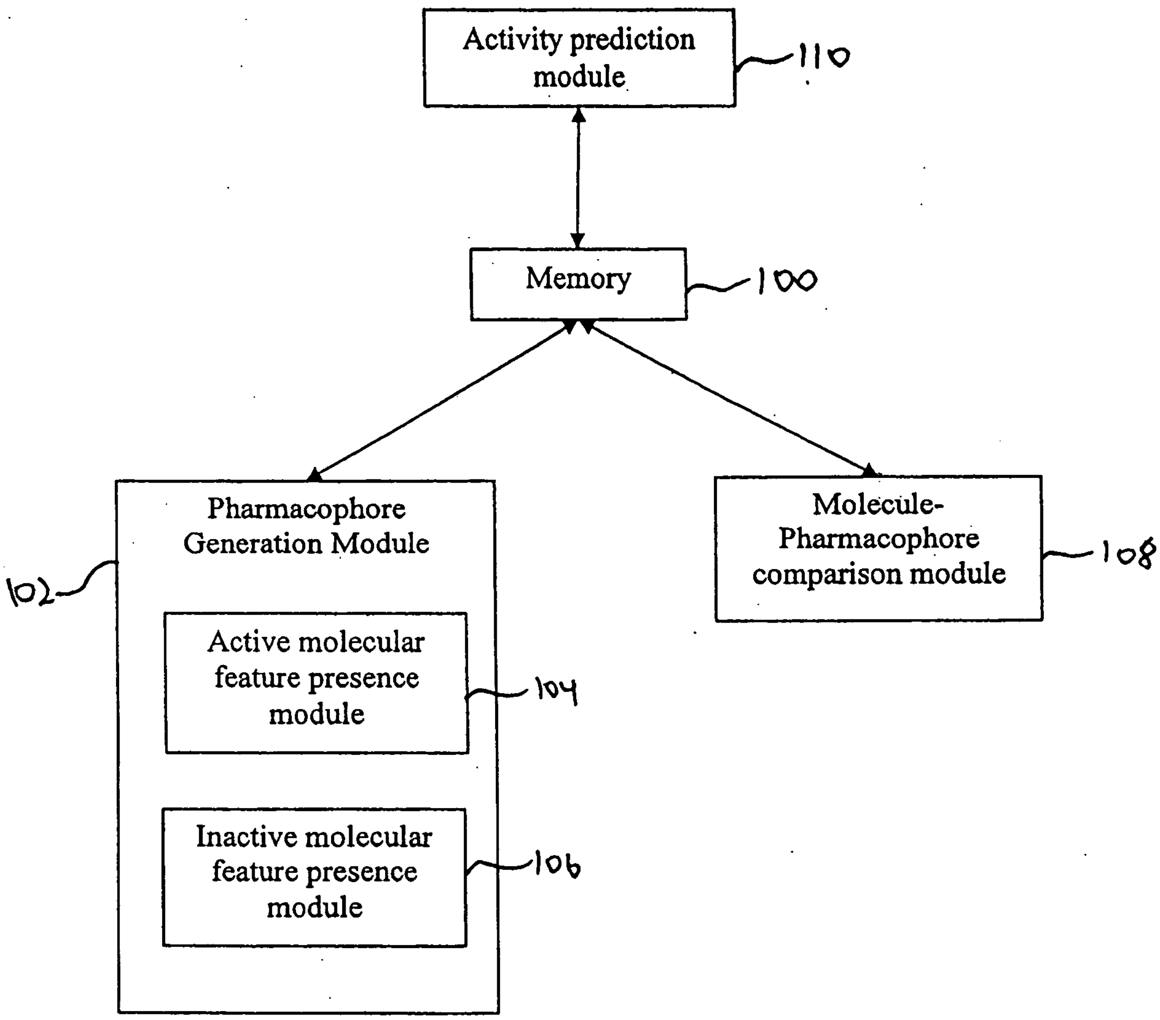
Activity prediction module
110
Memory
100
Pharmacophore Generation Module
102
Active molecular feature presence module
104
Inactive molecular feature presence module
106
Molecule-Pharmacophore comparison module
108

FIG. 3